# EUROPEAN PATENT SPECIFICATION

(11) **EP 2 982 412 B1**
(45) Date of publication and mention of the grant of the patent: **01.11.2017**
(21) Application number: 14778838.4
(22) Date of filing: 28.03.2014
(51) Int. Cl.: A61N 2/00, A61B 90/00, A61B 90/14, A61G 15/12

(54) **HEAD POSITIONING DEVICE, MEDICAL SYSTEM, MEDICAL INSTRUMENT POSITIONING DEVICE, AND HOLDING SHELL**
KOPFPOSITIONIERUNGSVORRICHTUNG, MEDIZINISCHES SYSTEM, VORRICHTUNG ZUR POSITIONIERUNG EINES MEDIZINISCHEN INSTRUMENTS UND HALTESCHALE
DISPOSITIF DE POSITIONNEMENT DE TÊTE, SYSTÈME MÉDICAL, DISPOSITIF DE POSITIONNEMENT D'INSTRUMENT MÉDICAL, ET COQUE DE MAINTIEN

(30) Priority: 02.04.2013 JP 2013077158
(43) Date of publication of application: 10.02.2016
(73) Proprietor: Teijin Pharma Limited, Tokyo 100-0013 (JP)
(72) Inventor: TAMIYA, Rei, Hino-shi Tokyo 191-0065 (JP); OKAYAMA, Takamitsu, Hino-shi Tokyo 191-0065 (JP); TOJO, Kenji, Hino-shi Tokyo 191-0065 (JP); MATSUI, Jun, Matsuyama-shi Ehime 791-8042 (JP)
(74) Representative: Hoffmann Eitle
(86) International application number: PCT/JP2014/059344
(87) International publication number: WO 2014/163021

(56) References cited:
- WO-A1-2012/059917
- JP-A- 2007 526 027
- JP-A- 2009 000 321
- JP-U- S 595 907
- US-A- 3 957 262
- US-A- 5 081 665
- US-A- 5 081 665
- US-A1- 2005 148 808
- US-A1- 2007 270 683
- US-A1- 2007 270 683
- US-B1- 7 024 237
- US-B2- 7 350 250
- US-B2- 7 350 250

## Description

### TECHNICAL FIELD

The present invention relates to a head positioning device, a medical system, a medical instrument positioning device, and a holding shell.

### BACKGROUND ART

Diagnosis or treatment of sickness or injury may require to position one or more of medical instruments on a body of a patient. Included is a transcranial magnetic stimulation (TMS) which is a non-invasive method of treatment of intractable disease such as nerve damage, for example. The transcranial magnetic stimulation is a method of treatment in which a symptom is ameliorated by giving a magnetic stimulation to a certain site (motor area) of the brain from the outside with a magnetic field generated by applying an electric current to a coil mounted on the head of a patient.

The transcranial magnetic stimulation requires the magnetic field to be applied on the motor area of the brain corresponding to a disease. The coil is desirably disposed in as close contact as possible with the patient in terms of energy efficiency. For example, Patent Document 1 describes that a magnetic stimulation device used in the transcranial magnetic stimulation has a coil fixed to a shell like a cap or a helmet to be placed on the head of a patient. The shell should always be placed at a predetermined position on the patient.

An appropriate position on which the medical instrument should be placed varies according to the symptom of the patient. Therefore, when using such shell described in Patent Document, it must be prepared for each patient or each symptom so that the shell can be held at a position to which the medical instrument should be fixed.

Patent Documents 2 and 3 describe methods of positioning a medical instrument to a desired part of a patient by disposing a measurement device measuring a relative position to a marker applied to a patient's body along with the medical instrument and by achieving a predetermined relation of the relative position of the measurement device to the marker. However, these methods need adjustments of the positions of the medical instrument and the measurement device, which in turn requires that a patient's head be kept immobile during the adjustment. This makes it extremely difficult for a patient to perform the adjustments by himself/herself.

Patent Document 4 describes an arrangement which measures a three-dimensional configuration of a human body, calculates a position at which the medical instrument should be disposed on the measured three-dimensional configuration, and positions a medical instrument. This arrangement, however, requires a measurement unit, a calculation circuit, and an actuator, which results in a significantly complicated system.

Patent document 5 describes a head restraint system.

### PRIOR ART DOCUMENT

### PATENT DOCUMENTS

WO 2007/123147 A1
JP 2003-180649 A
JP 2004-634 A
JP 2006-320425 A
US 2007/270683 A1

### SUMMARY OF THE INVENTION

### PROBLEM TO BE SOLVED BY THE INVENTION

In view of the above, the present invention is to provide a device having a simple system configuration capable of accurately disposing a medical instrument on a head of a user such as a patient.

### MEANS FOR SOLVING PROBLEM

The inventors conceived that a medical instrument can accurately be disposed relative to a head of a user by positioning and holding the medical instrument with a desired space from a head surface of the user, imaging a certain part of the user by an imaging means disposed in relation to the medical instrument, displaying a specified position at which the certain part should be located in an overlapping manner on the image, and allowing the user to move the held head by himself/herself within a range of the space so as to match the certain part to the specified position in the displayed image, and devised a head positioning device, a medical system, a medical instrument positioning device, and a holding shell for this purpose.

A head positioning device according to the present' invention is defined in claim 1.

According to this arrangement, at least one physical feature point is positioned by any one of the holding portions, the neck rest, and the head back rest and at least the movement of the cheeks or the neck of the user can be restricted. For example, if the user receives treatment in a sitting position, the holding portions cause the user to face forward and the height of the head can be determined and fixed relative to a positioned medical instrument. The neck rest can determine a position of the neck in the front-to-rear direction, i.e., a supporting point of the backward tilting of the head. The head back rest determines a position of the back of the head, i.e., an angle of the head. The above features enable correct regulation of the direction of the head relative to the medical instrument. As a result, the position and posture of the head of the user can substantially constantly be held before and/or during treatment. The posture of the user is not limited to the sitting posture described above and, for example, the posture before treatment (during positioning) and during treatment may be a standing position or a recumbent position (such as a dorsal position and a prone position). The posture may be different between during positioning and during treatment and, for example, the head positioning device is applicable to such a treatment instrument that is positioned in the standing position before treatment followed by the dorsal position with the positioning maintained.

In the head positioning device of the present invention, the holding portions supports a lower jaw or cheeks of the user from outside.

In the head positioning device of the present invention, a position of a physical feature point may be specified by at least a portion of this device. For example, the occipital protuberance of the user may be supported on the head back rest for positioning.

Since the user easily feels the contact pressure of the head back rest at the occipital protuberance, the contact pressure is made constant and the positioning accuracy is increased.

In the head positioning device of the present invention, the holding portions and the neck rest may integrally be formed into a U-shaped configuration.

The configuration including the neck rest and/or the holding portions holds the neck of the user in three directions and therefore produces an effect that the neck is favorably received while feeling of restraint is reduced. Since a user feels uncomfortable unless the device is correctly mounted, it is expected that the user's head is received each time at a correct position in the same way. Since the front side is opened, the user can immediately move away from the device without a release operation when some unexpected situation (such as a system defect and disaster) occurs.

The head positioning device of the present invention may further comprise a chair having a seat and a backrest, and the holding portions, the neck rest, and the head back rest may be fixed to the backrest. This configuration allows the user to take a comfortable posture and maintain the same posture for a long time. For example, if the user receives the treatment in a sitting position, the height of the neck is constant in accordance with the seating height and, therefore, the supporting point of inclination of the head determined by the neck rest is located each time at the same position. Even in other postures, the supporting point of the head can be adjusted each time to the same position. In other cases, at least one rest may be configured to be in contact with one physical feature point to maintain the same posture.

The head positioning device of the present invention can also have another positioning means so as to further increase the positioning accuracy during treatment. For example, the head positioning device may further comprise a camera for imaging a predetermined reference point on the head of the user, and a monitor for displaying an image taken by the camera and a position at which the reference point should be located. For another method of increasing the positioning accuracy, the device may include a monitor for displaying an image from the camera for imaging a relation between a predetermined reference point on the head of the user and a position at which the reference point should be located. In this case, the positions of the neck rest, the holding portions, and the head back rest for supporting the head can be changed in the vertical direction and left-to-right direction within ranges in which the functions are fulfilled, so as to enable the camera to image the predetermined reference point.

In the head positioning device of the present invention, the holding portions or the neck rest, preferably at least a surface portion thereof, is preferably formed of an elastic member. In this case, the high positioning accuracy can be maintained without giving feeling of restraint or pain to the user.

A medical system according to the present invention may have any of a plurality of the head positioning devices described above and a medical instrument positioning device for positioning a medical instrument used for treatment or diagnosis. Since this configuration keeps the head at a constant position with the head positioning device and keeps the medical instrument at a constant position with the medical instrument positioning device, the relative positions of the head and the medical instrument are made constant.

The medical system of the present invention has
the head positioning device, and
the medical instrument positioning device having an interface means between the head and the medical instrument, and
the medical instrument positioning device may have at least one movable mechanism for mounting the medical instrument and making the medical instrument movable before and during treatment.

For example, the interface means may be a holding shell brought close to, brought into contact with, or put on the head of the user along with the medial instrument mounted thereon. The interface means may be a winding means for holding wound around a portion of the head of the user or a means put around ears such as a head gear, a strap, a mask, and eyeglasses other than the holding shell.

The movable mechanism included in the medical instrument positioning device preferably enables the interface means to move on a virtual spherical surface covering the head, for example, along a virtual spherical surface around at least one point of the top of the head or a treatment part of the head. By way of example, the movable mechanism may be the following rail type movable mechanism.

A specific example of the rail type movable mechanism may be, for example, a movable mechanism rotationally moving along a first rail to tilt the holding shell and having a second rail for moving along the second rail extending in a direction different from the first rail to tilt the holding shell (to operate the interface means at a certain position on the virtual spherical surface). The number of rails is not limited.

The medical instrument positioning device may be capable of moving and positioning the medical instrument to a desired position on the virtual spherical surface in any other way. The medical instrument positioning device may further have a restriction mechanism for making the medical instrument immovable from a specified position after movement. The restriction mechanism may be, for example, a stopper of the movable mechanism or a mechanism limiting the movement within a certain extent.

With this configuration, the interface means may be tilted at any angle to adjust a relative position of the medical instrument to the head of the user.

In the medical system of the present invention, the first rail and the second rail may extend above the holding shell, keeping a constant distance from the interface means. With this configuration, a distance between the head surface and the interface means can be kept constant.

In the medical system of the present invention, the interface means may include a plurality of holding portions each capable of holding the medical instrument. With this configuration, an attachment position of the medical instrument to the interface means can be changed to enable treatment or diagnosis at different parts of the user.

As described above, the present invention provides the medical instrument positioning device having the interface means put on the head of the user along with the medical instrument used for treatment or diagnosis mounted thereon as well as the support mechanism and movable mechanism described above.

### EFFECT OF THE INVENTION

As described above, the present invention enables the positioning of both the user's head and the medical instrument.

### BRIEF DESCRIPTION OF DRAWINGS

Fig. 1 is a schematic view of a treatment system according to an embodiment of the present invention.
Fig. 2 is a detailed perspective view of a head positioning device of Fig. 1.
Fig. 3 is a perspective view of holding portions and a neck rest.
Fig. 4 is a schematic side view of a principle of positioning by the head positioning device of Fig. 2.
Fig. 5 is a schematic side view of an alternative configuration of the head positioning device of the present invention.
Fig. 6 is a perspective view of an embodiment of a medical instrument positioning device of the present invention.
Fig. 7 is a simplified side view for explaining a geometric shape of a holding shell of Fig. 2.
Fig. 8 is a perspective view of a holding shell applicable to the head positioning device of the present invention.
Fig. 9 is a perspective view of a state in which an exciting coil is mounted on the holding shell of Fig. 8.
Fig. 10 is a perspective view of an embodiment of a head back rest including a head back rest adjustment mechanism.

### DESCRIPTION OF THE INVENTION

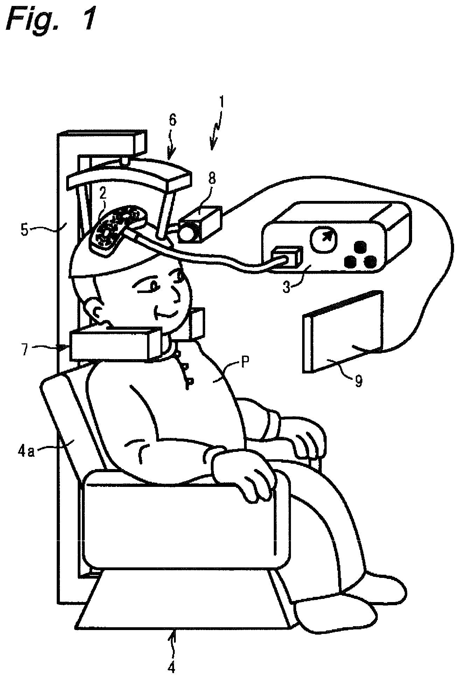

An embodiment of the present invention will now be described with reference to the drawings. First, Fig. 1 schematically depicts a basic configuration of a transcranial magnetic stimulation (TMS) system 1 according to an embodiment of a medical system of the present invention. The transcranial magnetic stimulation system 1 is a system which supplies electric power from a drive device 3 to a coil 2 adjacent the head of the user P to generate a magnetic field around the coil 2, thereby magnetically stimulating the brain of a user P.

The transcranial magnetic stimulation system 1 has a chair 4 on which the user P sits, a supporting member 5 fixed to the chair 4, a medical instrument positioning device 6 held by the supporting member 5 for positioning the coil 2, and a head positioning device 7 fixed to a backrest 4a of the chair 4 for positioning the head of the user P. The medical instrument positioning device 6 includes a camera 8 for imaging the head of the user P and a monitor 9 for displaying an image taken by

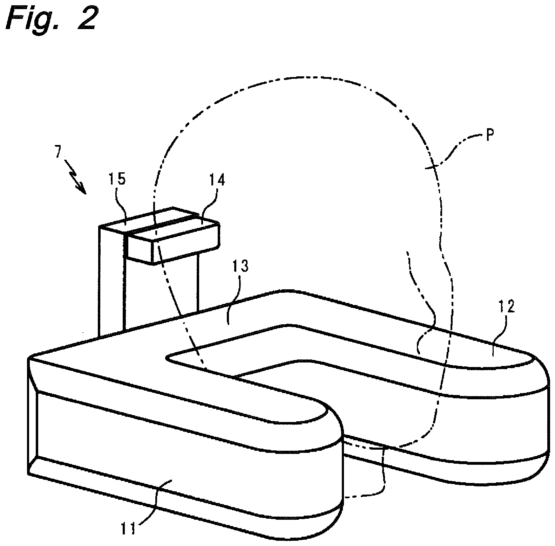

Fig. 2 depicts a detailed configuration of the head positioning device 7. The head positioning device 7 includes a pair of holding portions 11, 12 for holding a lower part of the head i.e. the cheeks or the lower jaw, of the user P from his or her left and right sides, a neck rest 13 for supporting a back portion of the neck of the user P, and a head back rest 14 for supporting the occipital protuberance of the user P. The holding portions 11, 12, the neck rest 13, and the head back rest 14 are preferably supported by a fixed member 15. The fixed member is preferably attached to the backrest 4a of the chair 4 or the supporting member 5, for example. The fixed member 15 may be eliminated if there is a replacement member capable of functioning in the same way, e.g., where the head back rest 14 is attached to the neck rest 13.

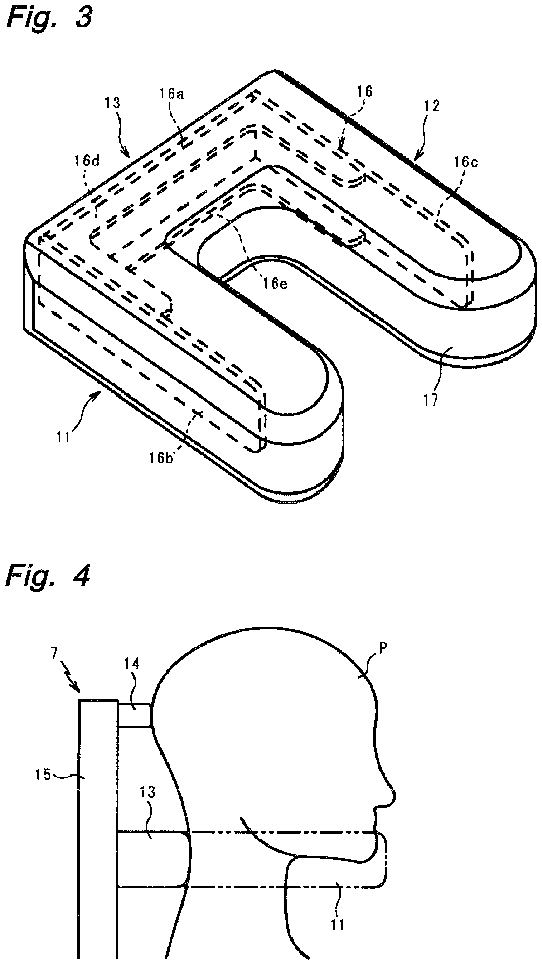

The holding portions 11, 12 and the neck rest 13 are formed integrally (eventually into a single piece), forming a U-shaped configuration. The holding portions 11, 12 and the neck rest 13 may not have the same height as depicted in Fig. 2, namely, they may have different heights. More specifically, as depicted in Fig. 3, the holding portions 11, 12 and the neck rest 13 are preferably formed of a U-shaped internal frame 16 made by assembling metal plates, a cushioning material (not depicted) such as urethane foam disposed to around the internal frame 16, and a cover 17 made from a material such as fabric and leather and covering the cushioning material.

The internal frame 16 has a base portion 16a acting as a core member of the neck rest 13, arm portions 16b, 16c extending forward from opposite ends of the base portion 16a and acting as core members of the holding portions 11, 12, and reinforcing portions 16d, 16e attached to internal upper and lower ends of the base portion 16a and the arm portions 16b, 16c to reinforce the U-shaped internal frame 16. The reinforcing portions 16d, 16e terminate at approximately mid portions of the arm portions 16b, 16c, spaced away from the distal ends of the arm portions 16b, 16c, increasing a deformability of the distal sides of the holding portions 11, 12. For the connection of the base portion 16a to the fixed member 15, no cushioning material is provided on the rear side of the base portion 16a.

The internal frame may be eliminated from the holding portions 11, 12 and/or the neck rest 13 provided each is capable of holding user's head immobile while maintaining its shape. For this reason, portions of the holding portions 11, 12 and/or the neck rest 13 which will be in contact with the user's head is preferably made of an elastic material rather than a hard material so that, when in use, the user does not suffer from painful or uncomfortable feeling.

An operation of the head positioning device 7 will be described with reference to Fig. 4. The user P sits on the chair 4 and leans the upper body against the backrest 4a. Then, the user P preferably moves his or her neck into a space between the holding portions 11, 12. During this motion, the holding portions 11, 12 deform due to the elasticity of the cushion material to accommodate the user's lower jaw therebetween, thereby holding the lower jaw from the opposite sides and supporting the lower jaw from its below. This results in that the user P is unable to rotate his or her head in left-to-right or nod by tilting his or her head forward.

Then, when the portion of the user's neck abuts on the neck rest 13, it is properly positioned with respect to the front-to-rear direction. The height of the user's neck is determined by the seating height of the user. Also, the neck rest 13 defines a supporting point for fixing the head of the user P. In order for a number of users to use the same system, the height of the fixed member 15 relative to the backrest 4a, for example, may be adjustable by using one or more screws or pins provided for one of the neck rest 13 and the fixed member 15 and one or more associated screw-holes or pin-holes provided for the other of the neck rest 13 and the fixed member 15. Of course, it is preferable that the neck rest 13 is maintained at a constant level to ensure that the same person can use the system repeatedly without any need to adjust the height of the neck rest 13.

The head back rest 14 preferably abuts on the occipital protuberance of the user P for the positioning of the head of the user P. The head back rest 14 supports the head of the user P from its back to prevent the head from tilting backward. The head back rest 14 may act to tilt the head of the user P forward around the supporting point defined by the neck rest 13. This allows the user's head to be held tightly with his or her lower jaw of the user P forcedly supported between the holding portions 11, 12.

Eventually, the head of the user P is held facing forward while maintaining a constant front-to-rear tilt angle. In this condition, however, since the holding portions 11, 12 have elasticity and the skin of the user P can move relative to his or her skull, the user P with the head held by the head positioning device 7 can slightly move his or her head.

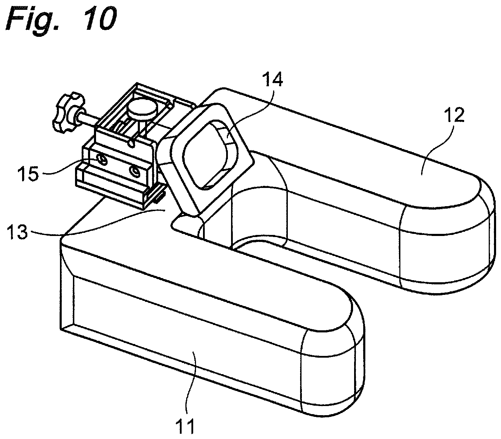

The shape of the occipital protuberance or the shape and convexity and concavity of the back of the head including or excluding the occipital protuberance varies from person to person. Then, as depicted in Fig. 10, a head back rest adjustment mechanism may be provided which allows the head back rest 14 to move in a front-to-rear direction or an up-and-down direction in order to move the contact point of the head. The head back rest may have a recessed or concave portion, or a protruded, expanded, or convex portion, corresponding to the configuration of the occipital protuberance or the back of the head. This allows the occipital protuberance or the back of the head to fit on the concave or convex portion, preventing a displacement of the occipital protuberance or the back of the head relative to the contacting head back rest. To ensure the occipital protuberance or the back of the user's head to make contacts at a fixed position, the head back rest may be tailor-made for each user.

According to the embodiment, the contact between the head back rest 14 and the occipital protuberance or the back of the head is reproduced at the same position, which ensures that a magnetic field can be applied to the same position as the previous treatment. Also, the stable reproduction of the contact between the head back rest and the occipital protuberance or the back of the head at the same position ensures that the magnetic field is maintained at the same position during a long time treatment of several tens of minutes or several hours, for example.

The transcranial magnetic stimulation system 1 uses the camera 9 to image an area including a predetermined reference point on the head of the user P, such as a mark made by art-makeup or a seal behind the ear and displays the image on the monitor 9. The monitor 9 displays the image captured by the camera 8 and also an indication or marker indicative of a position at which the reference point should be located when the head is properly positioned. The user P slightly moves the user's head to place the reference point on the indication within a permissible positional error which may be caused due to the elasticity of the head positioning device 7. This results in that the head of the user P is positioned and postured properly. A plurality of the cameras 8 may be provided to display images captured in different angles. The provision of the plural cameras ensures to obtain a large amount of positional information, ensuring a more accurate positioning.

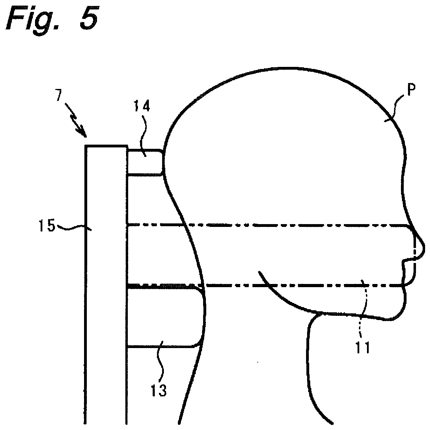

The holding portions 11, 12 may permit a certain amount of rotation and front-to-rear movement of the user's head. For example, as shown in in Fig. 5, the holding portions 11, 12 may be designed so that it supports the opposite lower cheek portions of the user P from their outside, which allows the user P can slightly open his or her mouth in this condition.

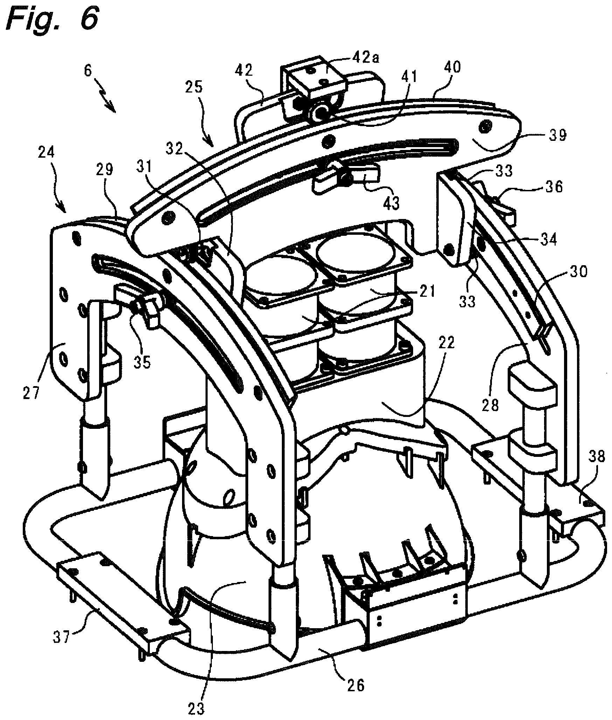

Fig. 6 depicts details of the medical instrument positioning device 6. The medical instrument positioning device 6 has a medical instrument assembly or magnetic head 22 including the coil 2 and having two exhaust fans 21, a holding shell 23 holding the magnetic head 22 and being designed to be placed on the head of the user P to act as an interface means between the user's head and the magnetic head 22, a first supporting mechanism 24 supporting the holding shell 23 so that it can tilt in the front-to-rear direction, and a second supporting mechanism 25 supporting the first supporting mechanism 24 so that it can tilt left-to-right direction. Although the holding shell 23 has a helmet structure covering the head of the user P in Fig. 6, the holding shell 23 may instead have a structure only mainly covering a bottom surface of the magnetic head 22.

The first supporting mechanism 24 includes a frame 26 holding the holding shell 23, a pair of arch portions 27, 28 arranged on the left and right sides of the user and extended upward from the frame 26, a pair of first rails 29, 30 attached respectively to the arch portions 27, 28, and supporting plates 32, 34 supporting slide rollers 31, 33 (depicted in part) engaged respectively with the first rails 29, 30. The first supporting mechanism 24 also has fixing screws 35, 36 capable of press-fixing the arch portions 27, 28 and the supporting plates 32, 34. Fixing tools 37, 38 are provided to the left and right portions of the frame 26 for holding the cameras 9 (not depicted in Fig. 6).

The second supporting mechanism 25 includes a connection plate 39 integrally holding the supporting plates 32, 34 of the first supporting mechanism 24, a second rail 40 attached to the connection plate 39, a slide roller 41 (depicted in part) engaged with the second rail 40, a fixing plate 42 supporting the slide roller 41, and a fixing screw 43 capable of press-fixing the connection plate 39 and the fixing plate 42. The fixing plate 42 has at its upper end a fixing unit 42a which is designed to be fixed to the supporting member 5 of Fig. 1.

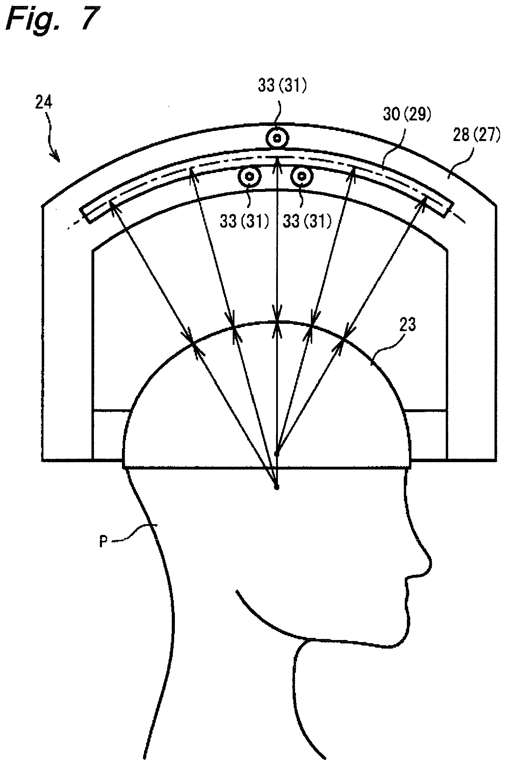

Fig. 7 depicts a relation between the holding shell 23 and the first rails 29, 30. In order to clearly depict the relation between the holding shell and the rails, one of two rails (i.e., rail 29) is eliminated from the drawing. The first rails 29, 30 are arranged symmetrically on the left and right sides of the frame 26. The holding shell 23 is constructed similar to the shape of the human head so that the holding shell 23 fits closely on the head of the user P. For example, in the front-to-rear cross section of the holding shell 23, as indicated at arrows in the drawing the front portion extending forward from the top of the head has a curvature which is smaller to that of the rear portion extending rearward from the top of the head. Correspondingly, the first rail 30 is constructed so that it has a front-to-rear cross section of which curvature varies with that of the holding shell 23, leaving a certain distance between front-to-rear extending lines of the first rail 30 and the holding shell 23 indicated at the arrows.

This ensures that, during the front-to-rear movement of the first rails 29 and 30 while being supported by the slide rollers 31, 33, a constant distance is maintained between the highest portion of the holding shell 23 and the slide rollers 31, 33 irrespective of the tilting angles of the holding shell 23 and the first rails 29, 30. Although three slide rollers 31, 33 are used in the embodiment in Fig. 7, the number of the slide rollers is not restrictive provided that the rails can slide on the rollers. Also, instead of slide rollers, another slide mechanism or slide rails may be used.

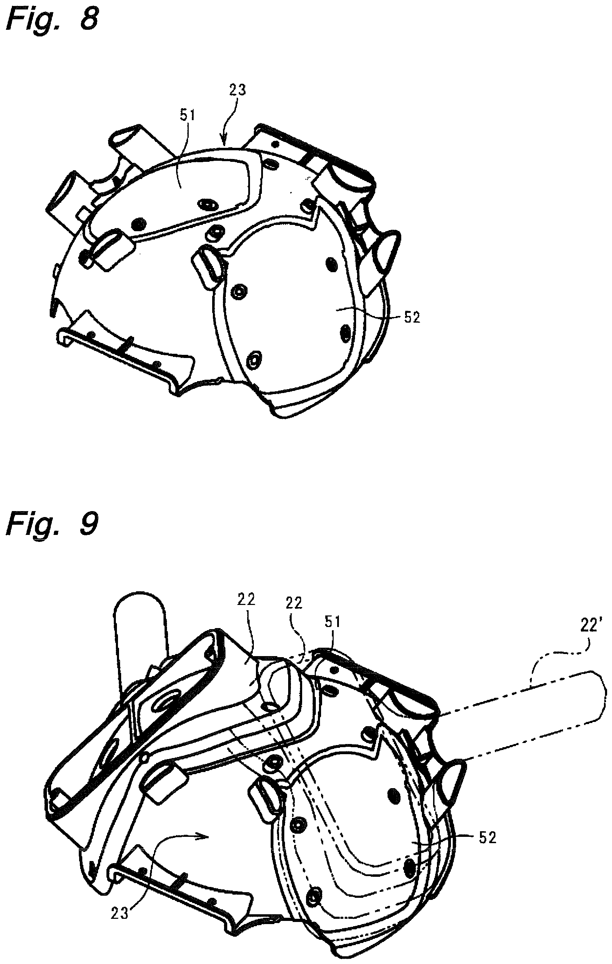

Although not depicted, the second rail 40 extends in a coronal plane to leave a certain distance between the second rail 40 and the holding shell 23. Although the external surface of the holding shell 23 has various uneven portions such as concave and convex portions (see Figs. 8 and 9), they should be neglected in the discussions of the relations between the holding shell 23 and the rails 29, 30, 40, and instead the discussions should be made in terms of the internal configuration of the holding shell.

With the configurations of the first rails 29, 30 and the second rail 40, a fitting condition of the holding shell 23 on the user P positioned by the head positioning device 7 is maintained substantially constant irrespective of the front-to-rear or left-to-right tilting angle of the holding shell 23. This means that the head positioning device 7 ensures an accurate selection and adjustment of the portion where the magnetic field generated by the coil 2 will be applied.

Scales or vernier scales may be provided to readily determine relative angles between the arch portions 27, 28 and the supporting plates 32, 34 and a relative angle between the connection plate 39 and the fixing plate 42, allowing the holding shell 23 to be adjusted at predetermined angles at each treatment, which in turn ensures an enhanced treatment effect.

When the magnetic coil 22 together with the holding shell 23 supporting the magnetic coil 22 is required to be moved from the top of the head to, for example, the temporal, occipital, or front portion of the head for moving the magnetic field irradiation site, it is tilted to a large extent and, even in this condition, the magnetic head 22 is needed to be held stably in the vicinity of the irradiation site. To this end, as shown in Fig. 8, preferably the holding shell 23 has one or more supporting portions 51, 52 in the form of, for example, recesses. Of course, the supporting positions correspond to the irradiation sites and therefore that the magnetic stimulation is applied to the aimed position in a reliable manner simply by mounting the magnetic head 22 at the associated supporting position.

In this embodiment, each of the supporting portions 51,52 may be designed to retain the magnetic head 22 with one portion (for example, one half) of the magnetic head 22 in close contact with the holding shell 23 and the remaining portion of the magnetic head 22 slightly away from the holding shell 23. With the arrangement, even when the magnetic stimulation is required to be applied to a treatment site which is positioned outside a zone surrounded by the holding shell, a part of the magnetic head is retained by the holding shell 23 at the supporting portion 51, 52 in a stable manner, allowing the magnetic stimulation to be applied to the treatment site.

In this embodiment, each of the supporting portions 51, 52 is designed so that only a portion of the magnetic head 22 is held in close contact with the holding shell 23 and the remaining portion is slightly spaced apart from the holding shell 23. This arrangement allows that, as depicted in Fig. 9, the magnetic heads 22 and 22' (indicated by solid and alternate long and two short dashed lines, respectively) are supported at the supporting portions 51, 52 and the floating (unfixed) portion of the magnetic head 22' extends over the magnetic head 22.

The number of the supporting portions is not restrictive and a plurality of supporting portions may be overlapped with each other. Evidently, the magnetic head supporting positions and the corresponding stimulating sites increase in proportion to the number of the supporting portions.

The shape and the position of the magnetic head 22 vary depending upon, in particular, the shape of the coil. Various shapes of coils are proposed such as a planar coil in which a conductive wire is extended spirally on a flat surface, a spherical coil in which the conductive wire is extended spirally on a spherical surface, and a special coil which is designed in consideration of, for example, treatment effect, power consumption, and convenience. Among others, one or more coils may be selected according to the treatment and mounted at respective proper positions. Although a conventional apparatus can apply the magnetic stimulation to a limited part, the transcranial magnetic stimulation system of the present invention enables selection from a larger number of parts to which the magnetic stimulation can be applied and, therefore, the magnetic coil can be disposed in a wider range. Thus, the magnetic stimulation can be applied to positions appropriate for various diseases and symptoms.

### PARTS LIST

- 1:: transcranial magnetic stimulation system (medical system)
- 2:: coil
- 4:: chair
- 4a:: backrest
- 5:: supporting member
- 6:: medical instrument positioning device
- 7:: head positioning device
- 8:: camera
- 9:: monitor
- 11, 12:: holding portion
- 13:: neck rest
- 14:: head back rest
- 15:: fixed member
- 16:: internal frame
- 17:: cover
- 22:: magnetic head (medical instrument)
- 23:: holding shell
- 24:: first supporting mechanism
- 25:: second supporting mechanism
- 29, 30:: first rail
- 31, 33:: slide roller
- 32, 34:: supporting plate
- 35, 36:: fixing screw
- 39:: connection plate
- 40:: second roller
- 41:: slide roller
- 42:: fixing plate
- 51, 52:: supporting portion

## Claims

1. A head positioning device comprising:
a pair of holding portions (11, 12) made of elastic material for supporting user's face from outside; a neck rest (13) for supporting a back of the neck of the user to determine a position of the neck in the front-to-rear direction; and
a head back rest (14) for supporting a back of the head to prevent the head from tilting backward, wherein: the pair of holding portions (11, 12) are arranged to restrict a frontward tilting of a head of the user and a rotation of a neck of the user, and the holding portions (11, 12) have elasticity and support a lower jaw or cheeks of the user from outside.

2. The head positioning device of claim 1, wherein the head back rest (14) supports an occipital protuberance of the user.

3. The head positioning device in claim 1 or 2, comprising a head rest adjustment mechanism for adjusting a position of the head back rest.

4. The head positioning device in any one of claims 1 to 3, wherein the head back rest (14) has a concave or convex portion formed therein corresponding to a shape of the occipital protuberance or the head back of the user.

5. The head positioning device in any one of claims 1 to 4, wherein the holding portions (11, 12) and the neck rest (13) are integrally formed into a U-shape configuration.

6. The head positioning device in any one of claims 1 to 5, further comprising a chair (4) having a seat and a backrest (4a), wherein the holding portions (11, 12), the neck rest (13), and the head back rest (14) are fixed to a retaining member which is held by a bracket.

7. The head positioning device in any one of claims 1 to 6, further comprising
a camera (8) for imaging a predetermined reference point on the head of the user, and
a monitor (9) for displaying an image captured by the camera (8) and a position at which the reference point should be located.

8. A medical system comprising:
the head positioning device in any one of claims 1 to 7; and
a medical instrument positioning device (6) for positioning a medical instrument used for treatment or diagnosis.

9. The medical system of claim 8, wherein
the medical instrument positioning device (6) has
a holding shell (23) which is placed on the head of the user, the holding shell (23) supporting a medical instrument mounted thereon,
a first supporting mechanism (24) for supporting the holding shell (23) and allowing the holding shell (23) to move along a first rail (29, 30) while changing a tilting of the holding shell (23),
and
a second supporting mechanism (25) for supporting the first supporting mechanism (24) and allowing the first supporting mechanism (24) to move along a second rail (40) extending in a direction different from that of the first rail (29, 30) to change a tilting of the first supporting mechanism (24).

10. The medical system of claim 9, wherein
the first rail (29, 30) and the second rail (40) extend above the holding shell (23) while leaving a constant distance from the holding shell (23).

11. The medical system of claim 9 or 10, wherein
the holding shell (23) includes a plurality of supporting portions (51, 52) each capable of supporting the medical instrument, and wherein
at least one of the supporting portions (51, 52) supports only a portion of the medical instrument so that a remaining portion of the medical instrument is positioned floatingly above at least one of other supporting portions (51, 52).

## Patentansprüche

1. Kopfpositionierungsvorrichtung, umfassend:
ein Paar von aus elastischem Material hergestellten Halteteilen (11, 12) zum Stützen des Gesichts des Benutzers von außen; eine Nackenstütze (13) zum Stützen einer Rückseite des Halses des Benutzers zur Festlegung einer Position des Halses in der Richtung von vorne nach hinten; und
eine Hinterkopfstütze (14) zum Stützen einer Rückseite des Kopfs, um ein nach hinten Neigen des Kopfs zu verhindern, wobei:
das Paar von Halteteilen (11, 12) dazu eingerichtet ist, ein Vorwärtsneigen eines Kopfs des Benutzers und ein Drehen eines Halses des Benutzers einzuschränken, und
die Halteteile (11, 12) über Elastizität verfügen und einen Unterkiefer oder Wangen des Benutzers von außen stützen.

2. Kopfpositionierungsvorrichtung nach Anspruch 1, wobei die Hinterkopfstütze (14) einen Hinterhauptfortsatz des Benutzers stützt.

3. Kopfpositionierungsvorrichtung nach Anspruch 1 oder 2, umfassend einen Kopfstützenverstellmechanismus zum Verstellen einer Position der Hinterkopfstütze.

4. Kopfpositionierungsvorrichtung nach einem der Ansprüche 1 bis 3, wobei die Hinterkopfstütze (14) einen darin gebildeten konkaven oder konvexen Bereich aufweist, der einer Form des Hinterhauptfortsatzes oder des Hinterkopfs des Benutzers entspricht.

5. Kopfpositionierungsvorrichtung nach einem der Ansprüche 1 bis 4, wobei die Halteteile (11, 12) und die Nackenstütze (13) einstückig als eine U-förmige Konfiguration ausgebildet sind.

6. Kopfpositionierungsvorrichtung nach einem der Ansprüche 1 bis 5, weiter einen Stuhl (4) umfassend, der einen Sitz und eine Rückenlehne (4a) aufweist, wobei die Halteteile (11, 12), die Nackenstütze (13) und die Hinterkopfstütze (14) an einem Halteelement befestigt sind, das von einem Bügel gehalten wird.

7. Kopfpositionierungsvorrichtung nach einem der Ansprüche 1 bis 6, weiter umfassend
eine Kamera (8) zur Abbildung eines vorgegebenen Referenzpunkts auf dem Kopf des Benutzers, und
einen Bildschirm (9) zur Wiedergabe eines von der Kamera (8) aufgenommenen Bildes und einer Position, an der sich der Referenzpunkt befinden sollte.

8. Medizinisches System, umfassend:
die Kopfpositionierungsvorrichtung nach einem der Ansprüche 1 bis 7; und
eine Positionierungsvorrichtung (6) für ein medizinisches Instrument zur Positionierung eines zur Behandlung oder Diagnose verwendeten medizinischen Instruments.

9. Medizinisches System nach Anspruch 8, wobei
die Positionierungsvorrichtung (6) für ein medizinisches Instrument aufweist
eine Halteschale (23), die auf den Kopf des Benutzers aufgesetzt wird, wobei die Halteschale (23) ein daran montiertes medizinisches Instrument trägt,
einen ersten Stützmechanismus (24), um die Halteschale (23) zu stützen und zuzulassen, dass die Halteschale (23) sich entlang einer ersten Schiene (29, 30) bewegt, während eine Neigung der Halteschale (23) verändert wird,
und
einen zweiten Stützmechanismus (25), um den ersten Stützmechanismus (24) zu stützen und zuzulassen, dass der erste Stützmechanismus (24) sich entlang einer zweiten Schiene (40) bewegt, die sich in einer von der der ersten Schiene (29, 30) verschiedenen Richtung erstreckt, um eine Neigung des ersten Stützmechanismus (24) zu verändern.

10. Medizinisches System nach Anspruch 9, wobei
die erste Schiene (29, 30) und die zweite Schiene (40) sich über der Halteschale (23) erstrecken, während sie einen konstanten Abstand zu der Halteschale (23) einhalten.

11. Medizinisches System nach Anspruch 9 oder 10, wobei
die Halteschale (23) eine Vielzahl von Stützteilen (51, 52) beinhaltet, die jedes fähig sind, das medizinische Instrument zu stützen, und wobei
mindestens eines der Stützteile (51, 52) nur einen Teil des medizinischen Instruments stützt, sodass ein restlicher Teil des medizinischen Instruments schwebend über mindestens einem von anderen Stützteilen (51, 52) positioniert ist.

## Revendications

1. Dispositif de positionnement de tête comprenant :
une paire de parties de maintien (11, 12) réalisées avec un matériau élastique pour supporter le visage de l'utilisateur depuis l'extérieur ;
un repose-cou (13) pour supporter la nuque de l'utilisateur afin de déterminer la position du cou dans la direction avant - arrière ; et
un repose-tête (14) pour supporter l'arrière de la tête afin d'empêcher l'inclinaison de la tête vers l'arrière, dans lequel :
la paire de parties de maintien (11, 12) est agencée pour limiter une inclinaison vers l'avant de la tête de l'utilisateur et une rotation du cou de l'utilisateur, et
les parties de maintien (11, 12) présentent une élasticité et supportent une mâchoire inférieure ou les joues de l'utilisateur depuis l'extérieur.

2. Dispositif de positionnement de tête selon la revendication 1, dans lequel le repose-tête (14) supporte une protubérance occipitale de l'utilisateur.

3. Dispositif de positionnement de tête selon la revendication 1 ou 2, comprenant un mécanisme d'ajustement de repose tête pour ajuster une position du repose-tête.

4. Dispositif de positionnement de tête selon l'une quelconque des revendications 1 à 3, dans lequel le repose-tête (14) a une partie concave ou convexe formée à l'intérieur de ce dernier correspondant à une forme de la protubérance occipitale ou de l'arrière de la tête de l'utilisateur.

5. Dispositif de positionnement de tête selon l'une quelconque des revendications 1 à 4, dans lequel les parties de maintien (11, 12) et le repose-cou (13) sont formés de manière solidaire selon une configuration en forme de U.

6. Dispositif de positionnement de tête selon l'une quelconque des revendications 1 à 5, comprenant en outre un fauteuil (4) ayant un siège et un dossier (4a), dans lequel les parties de maintien (11, 12), le repose-cou (13) et le repose-tête (14) sont fixés à un élément de retenue qui est maintenu par un support.

7. Dispositif de positionnement de tête selon l'une quelconque des revendications 1 à 6, comprenant en outre :
une caméra (8) pour imager un point de référence prédéterminé sur la tête de l'utilisateur, et
un écran (9) pour afficher une image prise par la caméra (8) et une position dans laquelle le point de référence doit être positionné.

8. Système médical comprenant :
le dispositif de positionnement de tête selon l'une quelconque des revendications 1 à 7 ; et
un dispositif de positionnement d'instrument médical (6) pour positionner un instrument médical utilisé pour le traitement ou le diagnostic.

9. Système médical selon la revendication 8, dans lequel :
le dispositif de positionnement d'instrument médical (6) a :
une coque de maintien (23) qui est placée sur la tête de l'utilisateur, la coque de maintien (23) supportant un instrument médical monté sur cette dernière,
un premier mécanisme de support (24) pour supporter la coque de maintien (23) et permettre à la coque de maintien (23) de se déplacer le long du premier rail (29, 30) tout en changeant une inclinaison de la coque de maintien (23), et
un second mécanisme de support (25) pour supporter le premier mécanisme de support (24) et permettre au premier mécanisme de support (24) de se déplacer le long d'un second rail (40) s'étendant dans une direction différente de celle du premier rail (29, 30) pour changer une inclinaison du premier mécanisme de support (24).

10. Système médical selon la revendication 9, dans lequel :
le premier rail (29, 30) et le second rail (40) s'étendent au-dessus de la coque de maintien (23) tout en laissant une distance constante par rapport à la coque de maintien (23).

11. Système médical selon la revendication 9 ou 10, dans lequel :
la coque de maintien (23) comprend une pluralité de parties de support (51, 52), chacune capable de supporter un instrument médical, et dans lequel :
au moins l'une des parties de support (51, 52) ne supporte qu'une partie de l'instrument médical de sorte qu'une partie résiduelle de l'instrument médical est positionnée, de manière flottante, au-dessus d'au moins l'une des autres parties de support (51, 52).
